## Europäisches Patentamt

## European Patent Office

## Office européen des brevets

(19)

(11) Veröffentlichungsnummer : **0 143 895**
**B1**

(12)

# EUROPÄISCHE PATENTSCHRIFT

(45) Veröffentlichungstag der Patentschrift :
23.12.87

(21) Anmeldenummer : 84109715.7

(22) Anmeldetag : 16.08.84

(51) Int. Cl.⁴ : **A 61 M 5/14**, A 61 M 5/20, A 61 M 5/315

(54) Tragbares Infusionsgerät.

(30) Priorität : 07.09.83 CH 4887/83

(43) Veröffentlichungstag der Anmeldung :
12.06.85 Patentblatt 85/24

(45) Bekanntmachung des Hinweises auf die Patenterteilung : 23.12.87 Patentblatt 87/52

(84) Benannte Vertragsstaaten :
CH DE FR GB IT LI

(56) Entgegenhaltungen :
EP-A- 0 042 282
EP-A- 0 058 536
GB-A- 2 094 628
US-A- 3 886 938
US-A- 4 300 554

(73) Patentinhaber : DISETRONIC AG
Brunnmattstrasse 6
CH-3400 Burgdorf (CH)

(72) Erfinder : Michel, Peter
Blattnerweg 10
CH-3400 Burgdorf (CH)

(74) Vertreter : Keller, René, Dr. et al
Patentanwälte Hartmut Keller, Dr. René Keller Postfach 12
CH-3000 Bern 7 (CH)

Jouve, 18, rue St-Denis, 75001 Paris, France

## Beschreibung

Die Erfindung betrifft ein tragbares Infusionsgerät zum automatischen Abgeben von Flüssigkeit gemäss dem ersten Teil des Patentanspruchs 1.

Ein Gerät dieser Art ist aus der GB-A-2 094 628 bekannt. Es hat ein Gehäuse mit abnehmbarem Deckel, in das eine Spritze so einsetzbar ist, dass ihr Auslassstück durch ein Loch in der Seitenwand des Gehäuses herausragt. Der Kopf der Kolbenstange der Spritze wird in einen Ansatz einer Mutter eines Schraubengetriebes eingesetzt, dessen Gewindestange mittels einer an einem auf ihr sitzenden Ritzel angreifenden Antriebsvorrichtung drehbar ist. Beim Drehen der Gewindestange wird die Kolbenstange der Spritze durch die Mutter vorgeschoben. Das Gerät kann nur mit einer vollständigen, eine Kolbenstange aufweisenden Spritze betrieben werden und die Mutter wird durch die Kolbenstange exzentrisch belastet, so dass die Antriebskraft eine erhebliche Reibung überwinden muss und der Vorschub infolge Verschleiss bzw. Verkanten störungsanfällig ist. Zudem ist das Zurückschrauben der Mutter zum Einsetzen einer neuen (vollen) Spritze zeitaufwendig.

Ein Infusionsgerät anderer Art ist aus der EP-A 42 282 bekannt. Die für das Gerät zu verwendenden Spritzen haben am hinteren Gehäuseende einen Flansch, der in Rillen an gegenüberliegenden Gehäusewänden des Geräts eingesetzt wird. Die Kolbenstange der Spritze wird auf ein am Boden des Gehäuses zwischen den gegenüberliegenden Wänden angeordnetes Ritzel einer Antriebsvorrichtung gelegt und mittels eines Niederdrückorgans auf das Ritzel gedrückt, damit dieses die aus Kunststoff bestehende, in Längsrichtung glatte Kolbenstange vorschieben kann. Das Gerät hat gegenüber anderen, z. B. aus der US-A-3 886 938 bekannten Geräten, bei denen im Gehäuse eine von einem Ritzel der Antriebsvorrichtung vorgeschobene Zahnstange angeordnet ist, die an die Kolbenstange anzukuppeln ist, den Vorteil, dass das Ankuppeln entfällt und deshalb das mit der Spritze ausgerüstete Gerät kürzer ist. Der bei den bekannten Geräten dieser Art grundsätzlich bereits bestehende Nachteil, dass ein hohes Drehmoment für den Vorschub der Stange erforderlich ist, und die Gefahr besteht, dass die Stange infolge des verhältnismässig hohen Gegendrucks nicht zuverlässig vorgeschoben wird, oder sogar zurückläuft, besteht bei dem aus der europäischen Patentanmeldung bekannten Gerät aber noch in erhöhtem Mass. Weil das Ritzel nicht an einer Zahnstange sondern an einer glatten Stange angreift, besteht zusätzlich Schlupf-Gefahr, wobei die Stange sogar über das Ritzel zurückrutschen kann.

Der Erfindung liegt die Aufgabe zugrunde, ein einfaches, mit einer Spritzampulle betreibbares, möglichst kurzes und kleines Gerät zu schaffen, das einen zuverlässigen Vorschub des Kolbens der Spritzampulle gewährleistet.

Die erfindungsgemässe Lösung dieser Aufgabe ist Gegenstand des kennzeichnenden Teils des Anspruchs 1.

Die Spritzampulle, mit der das Gerät zu betreiben ist, die aber nicht zum Gegenstand des Anspruchs 1 gehört, unterscheidet sich von einer gewöhnlichen Ampulle dadurch, dass sie für die Verwendung als Spritze am einen Ende mit einem dicht schliessenden Kolben versehen und für die Einführung einer Kolbenstange offen und am anderen Ende mit einem Auslassstück ausgerüstet ist. Von einer vollständigen Spritze unterscheidet sie sich dadurch, dass sie keine Kolbenstange aufweist. Sie wird auch als Karpulle bezeichnet.

Bevorzugte Ausführungsarten der Erfindung sind in den Patentansprüchen 2 bis 10 umschrieben. Der Anspruch 2 sowie die Ansprüche 3 und 4 lösen dabei zusätzlich die Aufgabe, die Spritzampulle vor Beschädigungen geschützt im Gehäuse anzuordnen, eine wasserdichte Ausführung des Geräts zu ermöglichen, und einen Katheter gegen Wegziehen gesichert an das Auslassstück der Spritzampulle anzuschliessen.

Im folgenden werden anhand der Zeichnung Ausführungsbeispiele der Erfindung näher erläutert. Es zeigen :

Fig. 1 einen Achsenlängsschnitt durch ein tragbares Infusionsgerät mit eingesetzter Spritzampulle,

Fig. 2 einen Querschnitt nach der Linie II-II in Fig. 1 in grösserem Massstab,

Fig. 3 einen Querschnitt nach der Linie III-III in Fig. 1,

Fig. 4 einen Achsenlängsschnitt durch eine Variante des Infusionsgeräts nach Fig. 1,

Fig. 5 eine aufgegliederte Darstellung des Infusionsgeräts nach Fig. 4, in kleinerem Massstab, und

Fig. 6 einen Schnitt nach der Linie VI-VI in Fig. 5, in grösserem Massstab.

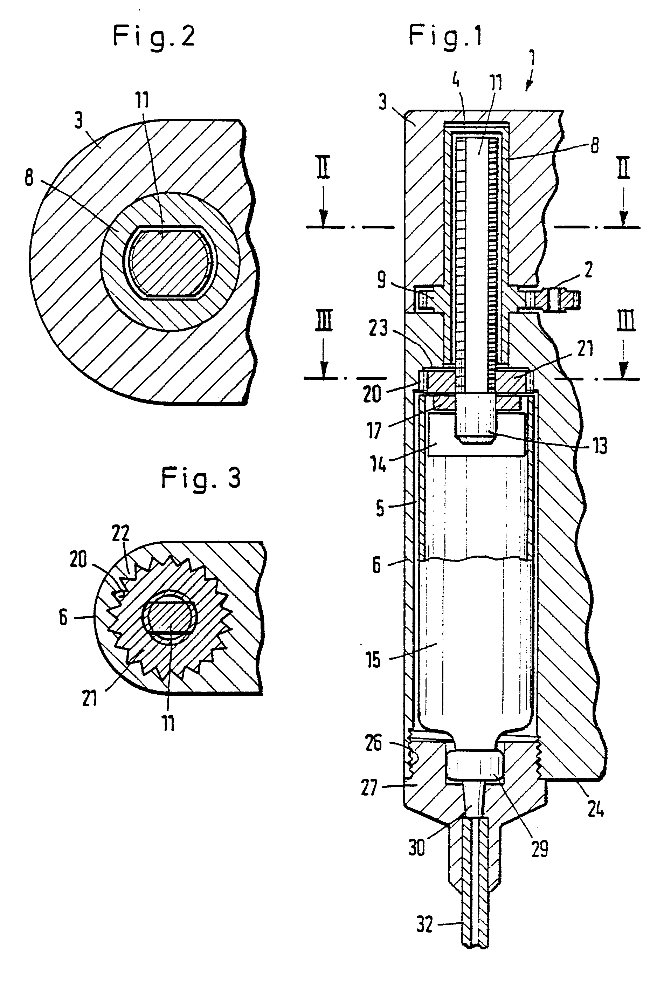

Das in Fig. 1 bis 3 dargestellte Gerät hat ein Gehäuse 1, in dem eine Antriebsvorrichtung angeordnet ist. Die (nicht dargestellte) Antriebsvorrichtung hat einen von einer Batterie gespeisten und durch eine Steuervorrichtung gesteuerten Schrittmotor, der ein Ritzel 2 antreibt. Die Steuervorrichtung kann die Taktfrequenz der den Schrittmotor steuernden Steuerimpulse im Tagesablauf nach einer vom Arzt festgelegten Funktion der Uhrzeit einstellen und dazu eine Uhr und einen Speicher aufweisen, in dem für bestimmte Uhrzeiten, z. B. jede Stunde des Tages, bestimmte, vom Arzt festgelegte Infusionsraten als Steuerwerte gespeichert sind.

Der hintere (in der Zeichnung obere) Teil 3 des Gehäuses 1 hat einen zylindrischen Hohlraum 4, der am hinteren Ende geschlossen ist und am vorderen Ende in einen zu ihm koaxialen, zylindrischen Hohlraum 5 des vorderen (in der Zeichnung unteren) Teils 6 des Gehäuses 1 mündet, dessen Hohlraumwand, wie weiter unten näher beschrieben, eine Halterung für eine auswechselbare Spritzampulle und eine Mutter bildet. Im Hohlraum 4 ist eine Mitnehmerhülse 8 drehbar gelagert, auf der ein in das Ritzel 2 greifender Zahnkranz 9 gebildet ist, der in einer ihm angepassten Aussparung des Gehäuseteils 3 läuft, wobei die Wände der Aussparung eine axiale Verschiebung des Zahnkranzes 9 und damit auch der Hülse 8 verhindern. (Um die Hülse 8 mit dem Zahnkranz 9 bei der Herstellung des Geräts einsetzen zu können, wird das Gehäuse 1 aus zwei Stücken zusammengesetzt, zwischen denen die Aussparung gebildet ist, und die nach Einsetzen der Hülse 8 zusammmgefügt, z. B. verklebt werden.) In die Mitnehmerhülse 8 ist eine Gewindestange 11 drehfest, aber längsverschiebbar eingesetzt. Für die drehfeste Lagerung ist die Gewindestange 11 an gegenüberliegenden Seiten abgeflacht und die Bohrung der Mitnehmerhülse im Querschnitt entsprechend angepasst. Die Gewindestange 11 hat also zwei ebene, parallele, glatte Längsflächen, an denen sie von den entsprechenden ebenen Innenwandflächen der Hülse 8 mitgenommen wird, und zwei mit dem Gewinde versehene Zylindermantelsegmente, die einen Abstand von den beiden glatten, zylindrischen Innenwandflächen der Hülse 8 haben. Am vorderen (in der Zeichnung unteren) Ende der Gewindestange 11 ist eine mit einem koaxial zur Gewindestange 11 vorstehenden, gewindelosen Zapfen 13, der in das für die Aufnahme einer Kolbenstange vorgesehene Sackloch des Kolbens 14 einer Spritzampulle 15 einführbar ist, gebildete Scheibe 17 drehbar gelagert. Der Durchmesser der Scheibe 17 ist kleiner als der Durchmesser des Kolbens 14, so dass die Scheibe auf dem Kolben 14 aufliegend in das Ampullenrohr einführbar ist. Auf den an die Scheibe 17 angrenzenden, in der Zeichnung unteren Teil der Gewindestange 11 ist eine mit einer Aussenverzahnung 20 versehene Mutter 21 geschraubt. Beim weiter unten beschriebenen Einsetzen der Gewindestange 11 in die Hülse 8 wird die Aussenverzahnung 20 der Mutter 21 in Eingriff gebracht mit einer entsprechenden Innenverzahnung 22 an der an die hintere Stirnwand 23 des zylindrischen Hohlraums 5 angrenzenden Mantelfläche der Innenwand des Gehäuseteils 6. Die Dicke der Mutter 21 ist grösser als die Zahnbreite der Innenverzahnung 22, und der Durchmesser des Kopfkreises der Aussenverzahnung 20 entspricht etwa dem Aussendurchmesser der Spritzampulle 15, so dass die Mutter 21 nach dem weiter unten beschriebenen Einsetzen der Spritzampulle 15 zwischen deren hinterem Ende und der hinteren Stirnwand 23 des Hohlraums 5 gegen axiale Verschiebung gesichert eingeklemmt ist. Das freie, vordere (in der Zeichnung untere) Ende 24 des Gehäuseteils 6 ist mit einem Innengewinde versehen, in das ein mit einem Aussengewinde 26 versehener Anschlussteil 27 geschraubt ist. Dieser hat einen zylindrischen Hohlraum für die Aufnahme der Aluminiumkappe 29 der Spritzampulle 15 und einen konischen Hohlraum für die Aufnahme des aus Kunststoff bestehenden Auslassstücks 30 der Spritzampulle 15, des sogenannten Luers. Beim Anschrauben des Anschlussteiles 27 wird der Luer 30 dicht an die Wandung des konischen Hohlraums gedrückt. In das vordere Ende des Anschlussteiles 27 ist ein mit dem konischen Hohlraum kommunizierender durch ein biegsames Röhrchen gebildeter Katheter 32 geschweisst, der bei dicht an den konischen Hohlraumwänden anliegendem Luer 30 an dessen Austrittsöffnung angrenzt oder einen kleinen Abstand von ihr hat.

Für das Gerät können handelsübliche Spritzampullen verwendet werden. Die in der Zeichnung dargestellte Spritzampulle 15 ist eine solche handelsübliche Spritzampulle, deren Kolben 14 ein Gewindesackloch für eine Kolbenstange hat. Bevorzugt werden bereits z. B. mit Insulin gefüllte Spritzampullen verwendet, die ohne Kolbenstange geliefert werden. Der Patient kann aber auch leere Spritzampullen verwenden, die mit einer in das Gewindesackloch schraubbaren handelsüblichen Kolbenstange geliefert werden. Nach dem Füllen der Spritzampulle hat der Patient dann lediglich die Kolbenstange aus dem Gewindesackloch herauszuschrauben.

Die gefüllte Spritzampulle 15 wird mit ihrem Luer 30 — nach Entfernen einer diesen verschliessenden Schutzkappe — in den Anschlussteil 27 gesteckt. Danach wird die Gewindestange 11, auf welcher die Mutter 21 anliegend an der Scheibe 17 sitzt, mit dem drehbar an ihr gelagerten Zapfen 13 in das Sackloch des Kolbens 14 gesteckt, so dass die Scheibe 17 am Kolben 14 anliegt. Nun wird die Spritzampulle 15 mit Hilfe des griffigen Anschlussteils 27 in den Hohlraum 5 eingeführt und nach hinten geschoben, wobei sie durch die zylindrische Hohlraumwandung geführt und die Gewindestange 11 koaxial an die Mitnehmerhülse 8 heran und in diese hinein geführt wird. Beim weiteren Vorschieben greift die Aussenverzahnung 20 der Mutter 21 in die Innenverzahnung 22. (Um das Einführen der Gewindestange 11 in die Mitnehmerhülse 8 zu erleichtern, kann das hintere Ende der Gewindestange verjüngt sein. Entsprechend kann die Aussenverzahnung 20 der Mutter 21 an der in die Innenverzahnung 22 einzuführenden Stirnseite abgeschrägt sein.) Schliesslich wird der Anschlussteil 27 mit seinem Gewinde 26 in das Gehäuseende 24 geschraubt. Beim Anschrauben drückt die an der Stirnwand 23 des Hohlraums 5 abgestützte Mutter 21 die über das Spritzampullenende vorstehende Scheibe 17 und damit auch den bündig im hinteren Ende der Spritzampulle sitzenden Kolben 14 nach vorne, so dass die Flüssigkeit aus der Spritzampulle 15 in den mit dem Luer 30 kommunizierenden Katheter 32 austritt. Die für den Vorschub des Kolbens 14 beim Anschrauben des Anschlussteils 27 massgebenden Abmessungen (Länge des Hohlraums 5, der Hohlräume im Anschlussteil 27, des Gewindes 26 usw.) sind so gewählt, dass die Flüssigkeit mit Sicherheit den Katheter bis hin zur Spitze der an ihn angeschlossenen Nadel füllt, d. h. eine geringe Flüssigkeitsmenge aus der Nadel austritt. Nach dem Anschrauben des Anschlussteils 27 liegt der hintere Rand des Hohlzylinders der Spritzampulle 15 an der Mutter 21 an, sodass die Mutter zwischen dem hinteren Spritzampullenrand und der Stirnwand 23 gegen axiale Verschiebung gesichert ist.

Das nun betriebsbereite Gerät arbeitet wie folgt : Der durch die Steuervorrichtung gesteuerte Motor treibt über das Ritzel 2 den an der Mitnehmerhülse 8 gebildeten Zahnkranz 9 an. Die Mitnehmerhülse 8 nimmt bei ihrer Drehung die Gewindestange 11 mit, die sich unter Vorschub des Kolbens 14 als Schraubenspindel durch die von der Verzahnung 22 drehfest im Gehäuse gehaltene Mutter 21 nach vorne schraubt.

Die drehbare Lagerung von Scheibe 17 und Zapfen 13 an der Gewindestange 11 hat gegenüber einer grundsätzlich selbstverständlich auch möglichen, festen Lagerung den Vorteil, dass keine Reibung am Kolben auftritt und deshalb der Stromverbrauch des Antriebsmotors klein gehalten wird. Es hat sich überraschenderweise gezeigt, dass diese drehbare Lagerung das Einführen der Gewindestange 11 in die Mitnehmerhülse 8 (und der Mutterverzahnung 20 in die Innenverzahnung 22) nicht erschwert sondern im Gegenteil gegenüber einer festen Lagerung eher erleichtert. Bei einer festen Lagerung ist der Anschlussteil 27 mit der Spritzampulle 15 beim Einführen so zu drehen, dass die Gewindestange 11 in die richtige Lage für die Einführung in die Mitnehmerhülse 8 gelangt. Bei der drehbaren Lagerung genügt eine leichte Rüttelbewegung, damit die Gewindestange 11 in die Mitnehmerhülse 8 hineinrutscht.

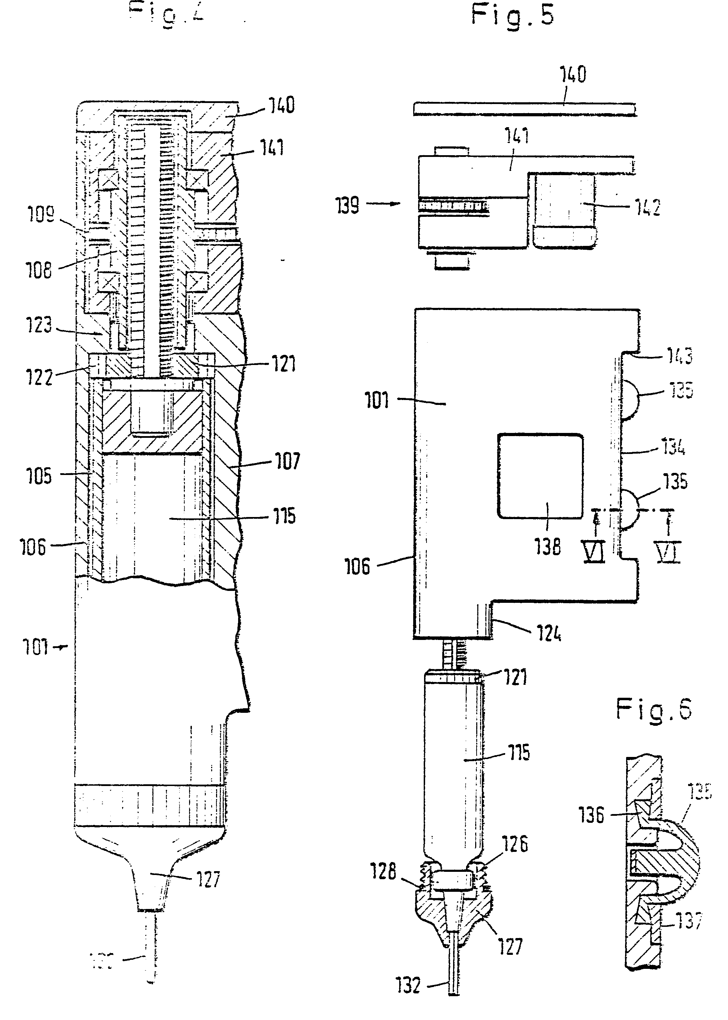

Die in Fig. 4-6 dargestellte Variante des Geräts ist im Prinzip gleich aufgebaut wie das Gerät von Fig. 1-3. Im folgenden werden deshalb im wesentlichen nur die Teile der Variante näher erläutert, die sich von den Teilen des Geräts gemäss Fig. 1-3 unterscheiden. Dabei sind die Teile der Variante, welche den im Zusammenhang mit Fig. 1-3 erwähnten Teilen 1, 2, 3, ..., in ihrer Funktion entsprechen, in Fig. 4-6, soweit sie mit Bezugszahlen versehen sind, mit 101, 102, 103, ..., bezeichnet.

Das Gehäuse 101 der dargestellten Variante hat eine als Halterung für die Spritzampulle 115 dienende, hohlzylindrische Kammer 105. Die Kammerwandung ist gebildet durch den vorderen (in der Zeichnung unteren) Teil der linken, halbzylindrischen Seitenwand 106 des Gehäuses 101, eine halbzylindrische Trennwand 107 und einen zylindrischen, mit einem Innengewinde versehenen Gehäuseansatz 124. Die Trennwand 107 erstreckt sich vom Gehäuseansatz 124 zu einer die Kammer 105 hinten (in der Zeichnung oben) begrenzenden Stirnwand 123, die ein der Mitnehmerhülse 108 angepasstes Loch aufweist und an der die Mutter 121 abgestützt wird. Die an die Stirnwand 123 angrenzende Mantelfläche der zylindrischen Kammerwandung weist die Innenverzahnung 122 auf. Vorne (in der Zeichnung unten) ist die Kammer 105 durch einen in den Gehäuseansatz 124 schraubbaren Anschlussteil 127 abschliessbar, an den ein Katheter 132 fest angeschlossen ist, der am anderen Ende einen (nicht dargestellten) zweiten Anschlussteil für die Nadel hat. Der zweite Anschlussteil ist ebenfalls fest mit dem Katheter verbunden, z. B. verschweisst, und mit einem Gewinde versehen, in das ein fest mit einer Nadel verbundener Gewindestutzen schraubbar ist. Damit ist eine gegen Zug gesicherte Verbindung von der Nadel über den Katheter bis zum Anschlussstück der Spritzampulle gewährleistet. Am Gewindestutzen 126 des Anschlussteils 127 ist ein O-Ring 128 vorgesehen, damit eine wasserdichte Verbindung des Anschlussteils 127 mit dem Gehäuseansatz 124 sichergestellt ist.

Im Raum zwischen der Trennwand 107 und der rechten Seitenwand 134 des Gehäuses 101 ist eine (nicht dargestellte) Steuervorrichtung für die Antriebsvorrichtung angeordnet. Die Steuervorrichtung hat zwei Schaltkontakte, die durch Druck auf zwei an der rechten Seitenwand 134 angeordnete Knöpfe 135 betätigbar sind (Fig. 6). Die Knöpfe bestehen aus elastischem Material und haben einen Kragen 136, der mittels eines Rings 137 an den Rand des Durchgangslochs für die Betätigung des Schaltkontakts gedrückt ist, so dass das Loch wasserdicht abgeschlossen ist. Durch ein in die Gehäusewand eingefügtes Fenster 138 ist eine Anzeige der Steuervorrichtung ablesbar.

Zum Einbau der Steuervorrichtung und einer die Mitnehmehülse 108 und die Antriebsvorrichtung aufweisenden Baueinheit 139 ist das Gehäuse 101 an der hinteren (in der Zeichnung oberen) Seite offen, wobei die Oeffnung nach dem Einbau durch einen Deckel 140 dicht verschlossen wird. Die Baueinheit 139 hat einen Träger 141, an dem die Mitnehmerhülse 108 drehbar gelagert ist, und der einen Schrittmotor 142 sowie ein Getriebe mit einen den Zahnkranz 109 der Mitnehmerhülse 108 antreibenden Ritzel trägt. Die in das Gehäuse 101 eingesetzte Baueinheit 139 ist zwischen der Stirnwand 123 sowie einem Gehäuseansatz 143 und dem Deckel 140 gehalten, wobei die Mitnehmerhülse 108 in das Loch der Stirnwand 123 und ein Sackloch im Deckel 140 hineinragt. Zur Speisung des Schrittmotors 142 und der Steuervorrichtung sind zwei separate (nicht dargestellte) Batterien vorgesehen, die in zwei durch Schraubverschlüsse dicht verschliessbaren, kleinen Kammern im vorderen (in Fig. 5 unteren) Teil des Gehäuses 101 zwischen der Kammer 105 und der rechten Seitenwand 134 angeordnet sind.

Durch die Erfindung wird ein zuverlässiges Vorschieben des Kolbens auch bei geringem Drehmoment der Antriebsvorrichtung (z. B. infolge nachlassender Batteriespannung) sichergestellt, wobei nur die für den Kolbenvorschub erforderliche axiale Kraft und keine radialen Kräfte auf die die Kolbenstange bildende Gewindestange wirkt : Die erfindungsgemässe Lösung ermöglicht es, die ganze Spritzampulle mit ihrem Anschlussstück sicher und geschützt im Gehäuse zu haltern. Dabei kann das Gehäuse sämtliche Teile des Geräts umschliessen und wasserdicht ausgeführt werden, so dass der Patient gefahrlos duschen oder sogar baden kann.

Durch die feste Verbindung des Katheters 32 mit dem Anschlussteil 27 wird die Gefahr vermieden, dass der Patient, z. B. im Schlaf, den Katheter wegreisst. Das erfindungsgemässe Gerät braucht nur gerade so lang bemessen zu werden, wie dies durch die Spritzampulle und die erforderliche Kolbenstangenlänge bedingt ist.

**Patentansprüche**

1. Tragbares Infusionsgerät zum automatischen Abgeben von Flüssigkeit, insbesondere Insulin, aus einer auswechselbaren Spritzampulle, mit einem mittels einer Antriebsvorrichtung (2 ; 142) antreibbaren, eine Gewindestange (11) und eine auf dieser sitzende Mutter (21 ; 121) aufweisenden Getriebe, dadurch gekennzeichnet, daß das Antriebsglied des Getriebes (8, 11, 21 ; 108, 11, 121) durch eine im Gehäuse (1 ; 101) des Gerätes drehbar gelagerte Mitnehmerhülse (8 ; 108) und das Abtriebsglied durch die Gewindestange (11) gebildet ist, daß die Mitnehmerhülse (8 ; 108) koaxial zu einer am Gehäuse (1 ; 101) des Geräts vorgesehenen Halterung (6, 27 ; 106, 107, 124, 127) für eine Spritzampulle (15 ; 115) angeordnet und die Gewindestange (11) koaxial in die Mitnehmerhülse (8 ; 108) einführbar ist, und daß die Mutter (21 ; 121) an einem an die Halterung (6, 27 ; 106, 107, 124, 127) angrenzenden Teil (22, 23 ; 122, 123) des Gehäuses (1 ; 101) gegen axiale Verschiebung abstützbar und drehfest halterbar und die Gewindestange (11) in der Mitnehmerhülse (8 ; 108) längsbeweglich und drehfest ist, damit die Gewindestange (11) für den unmittelbaren Vorschub des Kolbens (14) einer Spritzampulle (15 ; 115) durch Drehen der Mitnehmerhülse (8 ; 108) in die Halterung (6, 27 ; 106, 107, 124, 127) vorschiebbar ist.

2. Gerät nach Anspruch 1, dadurch gekennzeichnet, daß die Halterung (6, 27 ; 106, 107, 124, 127) aus zwei Teilen besteht, deren erster Teil (6 ; 106, 107, 124) in dem sämtliche übrigen Teile des Geräts umschliessenden Gehäuse (1 ; 101) gebildet ist, das eine in den ersten Halterungsteil (6 ; 106, 107, 124) mündende Öffnung zum Einführen einer Spritzampulle (15 ; 115) hat, und deren zweiter Teil durch einen die Öffnung abschliessenden, lösbar (26 ; 126) am Gehäuse (1 ; 101) befestigten Anschlussteil (27 ; 127) gebildet ist, der einen Hohlraum für die Aufnahme des Auslassstücks (30) einer Spritzampulle (15 ; 115) aufweist.

3. Gerät nach Anspruch 2, dadurch gekennzeichnet, dass an den Anschlussteil (27 ; 127) ein Katheter (32 ; 132) anschließbar ist, der mit dem Hohlraum des Anschlussteils (27 ; 127) kommuniziert.

4. Gerät nach Anspruch 3, dadurch gekennzeichnet, dass der Anschlussteil (27 ; 127) an das Gehäuse (1, 24 ; 101, 124) schraubbar (26 ; 126) und sein für die Aufnahme des Auslassstücks (30) einer Spritzampulle (15 ; 115) bestimmter Hohlraum konisch in Richtung auf den Katheter (32 ; 132) verjüngt ist, damit die Hohlraumwandung beim Anschrauben des Anschlussteils (27 ; 127) an das Gehäuse (1, 24 ; 101, 124) dicht an das Auslassstück (30) einer eingesetzten Spritzampulle (15, 115) anpressbar ist.

5. Gerät nach einem der Ansprüche 1 bis 4, dadurch gekennzeichnet, dass der an die Halterung (6, 27 ; 106, 107, 124, 127) angrenzende Teil (22, 23 ; 122, 123) des Gehäuses (1 ; 101) für das Haltern und Abstützen der Mutter (21 ; 121) eine der Umfangsfläche der Mutter (21 ; 121) angepasste, diese gegen Drehung sichernde Mantelfläche (22 ; 122) und eine ein Durchgangsloch für die Gewindestange (11) aufweisende Stirnwand (23 ; 123) hat, damit die Mutter (21 ; 121) bei einer in die Halterung (6, 27 ; 106, 107, 124, 127) eingesetzten Spritzampulle (15 ; 115) zwischen der Stirnwand (23 ; 123) und dem hinteren Ende der Spritzampulle (15 ; 115) gegen axiale Verschiebung gesichert gehalten ist.

6. Gerät nach Anspruch 5, dadurch gekennzeichnet, dass der Umfang der Mutter (21, 121) eine Aussenverzahnung (20) und die Mantelfläche eine entsprechende Innenverzahnung (22 ; 122) aufweist.

7. Gerät nach einem der Ansprüche 1 bis 6, gekennzeichnet durch eine drehbar am vorderen Ende der Gewindestange (11) gelagerte Scheibe (17), die bei einer in die Halterung (6, 27 ; 106, 107, 124, 127) eingesetzten Spritzampulle (15) an deren Kolben (14) anliegt.

8. Gerät nach Anspruch 7, dadurch gekennzeichnet, dass die Scheibe (17) einen koaxial zur Gewindestange (11) vorstehenden Zapfen (13) zum Einführen in ein Sackloch des Kolbens (14) einer Spritzampulle (15) hat.

9. Gerät nach einem der Ansprüche 1 bis 8, dadurch gekennzeichnet, dass auf der Mitnehmerhülse (8) ein Zahnkranz (9) sitzt oder gebildet ist, in den ein von der Antriebsvorrichtung angetriebenes Ritzel (2) greift.

10. Gerät nach einem der Ansprüche 1 bis 9, dadurch gekennzeichnet, dass die Gewindestange (11) zwei ebene, parallele Längsflächen hat, an denen sie von zwei entsprechenden, ebenen Innenwandflächen der Mitnehmerhülse (8) mitgenommen wird.

**Claims**

1. Portable infusion instrument for the automatic administering of a liquid, in particular insulin from a replaceable injection ampoule with a drive system which can be driven by means of a drive device (2 ; 142) and which is provided with a threaded rod (11) and a nut (21 ; 121) which is seated on this, characterised in that the drive member of the drive system (8, 11, 21 ; 108, 11, 121) is formed by a drive sleeve (8 ; 108) rotatably mounted in the casing (1 ; 101) of the instrument and the output member by the threaded rod (11), that the drive sleeve (8 ; 108) is arranged coaxially with a holder (6, 27 ; 106, 107, 124, 127) for an injection ampoule (15 ; 115) which is provided at the casing (1 ; 101) of the instrument and the threaded rod (11) can be introduced coaxially into the drive sleeve (8 ; 108) and that the nut (21 ; 121) is supported against axial movement and can be held so that it is resistant to rotation at a part (22, 23 ; 122, 123) of the casing (1 ; 101) bordering on the holder (6, 27 ; 106, 107, 124, 127) and the threaded rod (11) in the drive sleeve (8, 108) can be moved longitudinally and is resistant to rotation, so that the threaded rod (11) can be slid forward into the holder (6, 27 ; 106, 107, 124, 127) for the direct feeding movement of the piston (14) of an injection ampoule (15 ; 115) by rotating the drive sleeve (8 ; 108).

2. Instrument according to claim 1, characterised in that the holder (6, 27 ; 106, 107, 124, 127) consists of two parts, the first part (6 ; 106, 107, 124) of which is formed in the casing (1 ; 101) surrounding all the other parts of the instrument, which is provided with an opening for introducing an injection ampoule (15 ; 115) which opens out into the first holder part (6 ; 106, 107, 124) and the second part of which is formed by a connection part (27, 127) which closes the opening and which is secured at the casing (1 ; 101) so that it is detachable (26 ; 126) and which is also provided with a hollow space for accommodating the outlet piece (30) of an injection ampoule (15 ; 115).

3. Instrument according to claim 2, characterised in that a catheter (32 ; 132) can be connected to the connection part (27 ; 127), whereby the catheter communicates with the hollow space of the connection part (27 ; 127).

4. Instrument according to claim 3, characterised in that the connection part (27, 127) can be screwed (26 ; 126) to the casing (1, 24 ; 101, 124) and for accommodating the outlet piece (30) of an injection ampoule (15 ; 115) a certain hollow space is tapered in the direction of the catheter (32 ; 132) so that the wall of the hollow space can be pressed tightly against the outlet piece (30) of an inserted injection ampoule (15, 115) when the connection part (27 ; 127) is screwed on to the casing (1, 24 ; 101, 124).

5. Instrument according to one of claims 1 to 4, characterised in that the part (22, 23 ; 122, 123) of the casing (1 ; 101) for holding and supporting the nut (21 ; 121) which borders the holder (6, 27 ; 106, 107, 124, 127) has a shell surface (22 ; 122) which matches the circumferential surface of the nut (21 ; 121) and which prevents it from rotating and which also has a front wall (23 ; 123) provided with a passage hole for the threaded rod (11), so that the nut (21 ; 121) is held secured against axial movement between the front wall (23 ; 123) and the rear end of the injection ampoule (15 ; 115) with an injection ampoule (15 ; 115) inserted in the holder (6, 27 ; 106, 107, 124, 127).

6. Instrument according to claim 5, characterised in that the circumference of the nut (21, 121) is provided with external toothing (20) and the shell surface is provided with corresponding internal toothing (22 ; 122).

7. Instrument according to one of claims 1 to 6, which is characterised by a disk (17) which is rotatably mounted at the front end of the threaded rod (11) and which with an injection ampoule (15) inserted in the holder (6, 27 ; 106, 107, 124, 127) abuts at its piston (14).

8. Instrument according to claim 7, characterised in that the disk (17) is provided with a protruding pin (13) which is co-axial with the threaded rod (11) for inserting an injection ampoule (15) into a blind hole of the piston (14).

9. Instrument according to one of claims 1 to 8, characterised in that a gear rim (9) is seated on the drive sleeve (8) or formed on it and with which a pinion (2) driven by the drive device engages.

10. Instrument according to one of claims 1 to 9, characterised in that the threaded rod (11) is provided with two even parallel longitudinal surfaces, at which it is picked up by two corresponding, even inner wall surfaces of the drive sleeve (8).

## Revendications

1. Appareil de perfusion portatif pour délivrer automatiquement un fluide, en particulier de l'insuline à partir d'une ampoule d'injection interchangeable, présentant une transmission qui peut être menée au moyen d'un dispositif d'entraînement (2 ; 142) et qui comporte une tige filetée (11), ainsi qu'un écrou (21 ; 121) calé sur cette tige, caractérisé par le fait que l'organe menant de la transmission (8, 11, 21 ; 108, 11, 121) est formé par une douille d'entraînement (8 ; 108) montée à rotation dans le boîtier (1 ; 101) de l'appareil, l'organe mené étant formé par la tige filetée (11) ; par le fait que la douille d'entraînement (8 ; 108) est disposée coaxialement à une retenue (6, 27 ; 106, 107, 124, 127) prévue sur le boîtier (1 ; 101) de l'appareil, pour une ampoule d'injection (15 ; 115), et la tige filetée (11) peut être introduite coaxialement dans la douille d'entraînement (8 ; 108) ; et par le fait que l'écrou (21 ; 121) peut prendre appui sans aucune faculté de coulissement axial, et être retenu sans aucune faculté de rotation, sur une partie (22, 23 ; 122, 123) du boîtier (1 ; 101) attenante à la retenue (6, 27 ; 106, 107, 124, 127), et la tige filetée (11) est mobile longitudinalement sans aucune faculté de rotation dans la douille d'entraînement (8 ; 108), de telle sorte que la tige filetée (11) puisse être poussée vers l'avant dans la retenue (6, 27 ; 106, 107, 124, 127) par une rotation de la douille d'entraînement (8 ; 108), en vue de l'avance directe du piston (14) d'une ampoule d'injection (15 ; 115).

2. Appareil selon la revendication 1, caractérisé par le fait que la retenue (6, 27 ; 106, 107, 124, 127) se compose de deux parties, dont la première partie (6 ; 106, 107, 124) est formée dans le boîtier (1 ; 101) qui entoure toutes les autres parties de l'appareil et présente une ouverture, débouchant dans la première partie (6 ; 106, 107, 124) de la retenue, en vue de l'introduction d'une ampoule d'injection (15 ; 115), et dont la seconde partie est formée par une pièce de raccordement (27 ; 127) qui obture l'ouverture, est fixée amoviblement (26 ; 126) au boîtier (1 ; 101), et présente une cavité pour recevoir l'embout de sortie (30) d'une ampoule d'injection (15 ; 115).

3. Appareil selon la revendication 2, caractérisé par le fait qu'un cathéter (32 ; 132), raccordable à la pièce de raccordement (27 ; 127), communique avec la cavité de cette pièce de raccordement (27 ; 127).

4. Appareil selon la revendication 3, caractérisé par le fait que la pièce de raccordement (27 ; 127) peut être vissée (26 ; 126) au boîtier (1, 24 ; 101, 124) et sa cavité, destinée à recevoir l'embout de sortie (30) d'une ampoule d'injection (15 ; 115), décroît tronconiquement de section en direction du cathéter (32 ; 132), de telle sorte que, lors du vissage de la pièce de raccordement (27 ; 127) au boîtier (1, 24 ; 101, 124), la paroi de la cavité puisse être pressée de manière étanche contre l'embout de sortie (30) d'une ampoule d'injection incorporée (15, 115).

5. Appareil selon l'une des revendications 1 à 4, caractérisé par le fait que la partie (22, 23 ; 122, 123) du boîtier (1 ; 101) attenante à la retenue (6, 27 ; 106, 107, 124, 127) présente, pour la retenue et l'appui de l'écrou (21 ; 121), une surface périphérique (22 ; 122) qui est adaptée à la surface circonférentielle de l'écrou (21 ; 121) et empêche une rotation de ce dernier, ainsi qu'une paroi extrême (23 ; 123) percée d'un trou traversant destiné à la tige filetée (11), de telle sorte que, lorsqu'une ampoule d'injection (15 ; 115) est introduite dans la retenue (6, 27 ; 106, 107, 124, 127), l'écrou (21 ; 121) soit retenu, d'une manière empêchant son coulissement axial, entre la paroi extrême (23 ; 123) et l'extrémité postérieure de l'ampoule d'injection (15 ; 115).

6. Appareil selon la revendication 5, caractérisé par le fait que le pourtour de l'écrou (21, 121) présente une denture externe (20), et la surface périphérique comporte une denture interne correspondante (22 ; 122).

7. Appareil selon l'une des revendications 1 à 6, caractérisé par un disque (17) qui est monté rotatif à l'extrémité antérieure de la tige filetée (11) et qui, en présence d'une ampoule d'injection (15) introduite dans la retenue (6, 27 ; 106, 107, 124, 127), est appliqué contre le piston (14) de cette ampoule.

8. Appareil selon la revendication 7, caractérisé par le fait que le disque (17) présente un tenon (13) faisant saillie coaxialement à la tige filetée (11), et destiné à pénétrer dans un trou borgne du piston (14) d'une ampoule d'injection (15).

9. Appareil selon l'une des revendications 1 à 8, caractérisé par le fait qu'une couronne dentée (9) est implantée ou ménagée sur la douille d'entraînement (8), couronne dans laquelle engrène un pignon (2) mené par le dispositif d'entraînement.

10. Appareil selon l'une des revendications 1 à 9, caractérisé par le fait que la tige filetée (11) possède deux surfaces longitudinales planes et parallèles, sur lesquelles elle est entraînée par deux surfaces planes correspondantes de la paroi interne de la douille d'entraînement (8).

# Fig.2

# Fig.1

# Fig. 3

# Fig.4

# Fig.5

# Fig.6